# EUROPÄISCHE PATENTSCHRIFT

(11) **EP 2 617 727 B1**
(45) Veröffentlichungstag und Bekanntmachung des Hinweises auf die Patenterteilung: **11.10.2017**
(21) Anmeldenummer: 12152026.6
(22) Anmeldetag: 22.01.2012
(51) Int. Cl.: C07H 3/04, C13K 5/00

(54) **Verfahren zur Ausbeuteverbesserung bei der Gewinnung von im Wesentlichen mineralstofffreier Lactose aus Molke**
Process for improving yield in the recovery of lactose essentially free of minerals from whey
Procédé d'amélioration du rendement lors de la production de lactose essentiellement sans matière minérale à partir de petit-lait

(43) Veröffentlichungstag der Anmeldung: 24.07.2013
(73) Patentinhaber: DMK Deutsches Milchkontor GmbH, 28199 Bremen (DE)
(72) Erfinder: Eckhoff, Jürgen, 27404 Elsdorf (DE)
(74) Vertreter: Fabry, Bernd

(56) Entgegenhaltungen:
- WO-A1-02/50089
- GB-A- 1 575 089
- US-A- 2 584 158
- US-A- 4 202 909

## Beschreibung

### Gebiet der Erfindung

Die Erfindung befindet sich auf dem Gebiet der Verarbeitung von Milchprodukten und betrifft ein Verfahren zur Verbesserung der Ausbeute an Lactose aus Molke, die im Wesentlichen frei von unerwünschten Mineralstoffen ist.

### Stand der Technik

Lactose gehört zur Gruppe der Disaccharide und besteht aus den beiden Molekülen D-Galactose und D-Glucose, die über eine β-1,4-glycosidische Bindung verbunden sind.

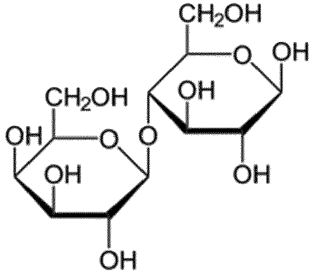

Lactose ist eine kristalline, farblose Substanz mit süßem Geschmack; die Süßkraft liegt je nach Konzentration zwischen 25 und 60 % der von Saccharose. Lactose stellt einen wichtigen Bestandteil der Milch dar und verfügt über eine Vielzahl von ernährungsphysiologischen Vorteilen. So dient es dem Metabolismus als Energiequelle, unterstützt die Calcium-resorption, hemmt die Bildung von Fäulnisbakterien im Darm und wirkt zumindest in größeren Mengen eingenommen, abführend. In der Lebensmitteltechnik wird es vor allem zur Herstellung von Milchsäure und als Texturiermittel für Tiefkühlprodukte eingesetzt. Da es Lebensmittel einen cremigen Geschmack verleiht, stellt es ein weit verbreitetes Additiv dar.

Lactose stellt ein Nebenprodukt bei der Herstellung von Proteinpulvern dar. Dabei wird üblicherweise Molke durch Ultrafiltration von Proteinen befreit, die anschließend einer Sprühtrocknung unterworfen werden.

Das bei der Filtration anfallende Permeat enthält im Wesentlichen Lactose sowie Mineralstoffe ("Asche") und Riboflavine. Um Lactose vermarkten zu können, ist es jedoch erforderlich, den Aschegehalt auf unter 0,3 Gew.-% zu reduzieren und auch Riboflavine möglichst abzutrennen, da diese für eine unerwünschte Gelbfärbung des Produktes verantwortlich sind. Für spezielle pharmazeutische Anwendungen ist es sogar erforderlich, den Aschegehalt auf unter 0,1 Gew.-% abzusenken.

Zur Herstellung einer solchen im Wesentlichen mineralstofffreien Lactose ist daher eine umfangreiche mehrstufige Aufarbeitung erforderlich. Aus der DE 2823244 A1 (Stauffer) ist beispielsweise bekannt, dass man Lactosekristalle mit wässriger Alkalilauge bei pH 8,5 bis 12,5 waschen kann, um die Farbqualität zu verbessern. In der Britischen Schrift GB 163937 (Martin) wird die Aufreinigung von Lactose mit Schwefeldioxid, Sulfiten oder Hydrogensulfiten vorgeschlagen. Von Yilmaz et al. wird ein Kristallisationsprozess für Lactose-Monohdyrat mit einem Aschegehalt von etwa 1 Gew.-% mittels wässrigem Ethanol beschrieben [Milchwissenschaft 52, S. 629-631 (1997**)**]. Die Demineralisierung von Permeat mit Hilfe von lonenaustauschern gefolgt von einer alkoholischen Kristallisation wird auch von Singh et al. beschrieben [J. Food Sci. 56, S. 777-788 (1991**)**]. Die Demineralisierung mittels einer Elektrolyse wird in EP 0315135 A2 (Valio) beschrieben.

Ein heute übliches Verfahren wird in der internationalen Patentanmeldung WO 2002 050089 A1 (Food Science Australia) beschrieben. Hierzu wird im ersten Verfahrensschritt das bei der Ultrafiltration der Molke gewonnene Permeat durch Umkehrosmose (RO) oder Nanofiltration (NF) aufkonzentriert. Anschließend wird das so gewonnene Konzentrat in zwei Schritten demineralisiert, d.h. zunächst mit einem Erdalkalisalz, üblicherweise einer wässrigen Calciumchloridlösung versetzt, wobei die Mineralien als Calciumphosphat gefällt werden. In der zweiten Fällungsstufe wird die Löslichkeit der Calciumsalze durch Zugabe von niederen Alkoholen weiter herabgesetzt und weiteres Phosphat gefällt. Die Abtrennung der Salze erfolgt dann mit Hilfe von geeigneten Filtrationsvorrichtungen, wie z.B. Membranen, Separatoren oder dergleichen. Anschließend wird die aufgereinigte Lactoselösung einer Vakuumdestillation unterworfen und auf einen Feststoffgehalt von etwa 65 Gew.-% eingestellt. Das Konzentrat wird in einem Kristallisator stark abgekühlt, die gebildeten Kristalle in einer Siebzentrifuge abdekantiert und dann auf einen Bandtrocknerabschließend entwässert.

Die bei der Kristallisation und anschließenden Dekantierung anfallende Mutterlauge, die auch als "Rework" bezeichnet wird, enthält jedoch noch etwa 30 Gew.-% Lactose. Ebenfalls enthalten sind jedoch auch noch lösliche Mineralstoffe, so dass die Lauge nicht ohne weitere Aufarbeitung weiterverarbeitet werden kann, um die darin enthaltene Lactose in zufriedenstellender Qualität zu gewinnen.

Verfahren zur Aufarbeitung der Mutterlauge mit dem Ziel der Rückgewinnung von Lactose sind grundsätzlich bekannt. So besteht natürlich grundsätzlich die Möglichkeit die Mutterlauge im Kreis zu führen und in den Demineralisierungsschritt einzuspeisen. Von Nachteil ist dabei allerdings, dass sich dabei der Restgehalt an löslicher Asche allmählich erhöht und sich die Qualität der Lactose in gleicher Weise nach und nach verschlechtert.

Aus dem Stand der Technik ist ein weiteres Verfahren bekannt, im dem vorgeschlagen wird, die Mutterlauge einer separaten Aufreinigung, z.B. durch Nanofiltration und anschließender Kristallisation zu unterwerfen. Bei diesem Verfahren müssen die Kristalle jedoch wieder in Wasser aufgelöst bzw. redispergiert werden, bevor die Lösung in die Demineralisierungsstufe aufgegeben und mit dem Hauptstrom vermischt wird. Auf diese Weise wird zwar die Lactoseausbeute gesteigert und ein Gesamtprodukt hoher Qualität erhalten, das Verfahren ist jedoch technisch aufwendig, so dass der ökonomische Nutzen zweifelhaft ist.

Aus der EP 0311977 B1 (Valio) ist ein Verfahren bekannt, bei dem man die Mutterlauge unter Einsatz eines speziellen sulfonierten Polystyrolharzes einer chromatographischen Aufreinigung unterzieht, bei der eine mineralstoffarme Lactosefraktion erhalten wird. Auch dieses Verfahren ist jedoch technisch zu aufwendig um realisiert zu werden.

Die Aufgabe der vorliegenden Erfindung hat somit darin bestanden, die Gesamtausbeute in dem eingangs beschriebenen Verfahren zur Herstellung einer im Wesentlichen mineralstofffreien Lactose aus Molkepermeat dahingehend zu verbessern, dass mit möglichst geringem Aufwand eine möglichst große Zusatzmenge an Lactose aus der Mutterlauge der Kristallisation gewonnen wird, die eine Qualität aufweist, die der der Hauptmenge des Verfahrens entweder gleichwertig ist oder wenigstens kaum nachsteht, so dass ein Verschneiden unter Einhaltung der Anforderung eines Mineralstoffgehaltes von nicht mehr als 0,3 Gew.-% und vorzugsweise unter 0,1 Gew.-% jederzeit möglich ist.

### Beschreibung der Erfindung

Gegenstand der Erfindung ist ein Verfahren zur Ausbeuteverbesserung bei der Gewinnung von im Wesentlichen mineralstofffreier Lactose, mit einem Mineralstoffanteil von weniger als 0.3 Gew.-%, bei dem man
(a) Molke einem Trennverfahren unterwirft, bei dem eine proteinreiche und eine lactosereiche Fraktion erhalten wird,
(b) die lactosereiche Fraktion gegebenenfalls nach Aufkonzentrierung (Hauptstrom) einer Demineralisierung unterwirft, bei der schwerlösliche Salze gefällt werden,
(c) den demineralisierten Rückstand gegebenenfalls nach weiterer Aufkonzentrierung abkühlt, bis Lactose kristallin ausfällt,
(d) die Lactosekristalle von der Mutterlauge abtrennt und entwässert, um eine erste Menge an Lactose zu erhalten,
und welches sich dadurch auszeichnet, dass man die Mutterlauge getrennt vom Hauptstrom einer Demineralisierung, Aufkonzentrierung, Abkühlung, Abtrennung der Kristalle und Trocknung unterwirft und die so gewonnene zweite Menge an Lactose mit der ersten Menge vermischt.

Überraschenderweise wurde gefunden, dass durch separate Aufarbeitung der Mutterlauge eine Lactosequalität erhalten wird, die der, die im Hauptverfahren gewonnen wird, nur unwesentlich nachsteht und problemlos mit der Hauptmenge vermischt werden. Auf diese Weise wird die Ausbeute bezogen auf die Menge an lactosehaltigem Einsatzmaterial erheblich verbessert, so dass das Verfahren ökonomisch günstiger durchgeführt werden kann. Gleichzeitig wird das Vorurteil überwunden, durch separate Aufarbeitung ließe sich analog zu den Erkenntnissen bei der Rückführung der Mutterlauge in die Demineralisierungstufe nur eine minderwertige Lactosequalität erhalten, die mit dem Hauptprodukt nicht verschnitten werden könne.

### Halb-Kontinuierliches Verfahren zur Lactosegewinnung

Die Gewinnung von Lactose erfolgt nach den Verfahren des Stands der Technik üblicherweise in einem kontinuierlichen Verfahren, das die folgenden Schritte (a) bis (d) umfasst:
(a) Molke einem Trennverfahren unterwirft, bei dem eine proteinreiche und eine lactosereiche Fraktion erhalten wird,
(b) die lactosereiche Fraktion gegebenenfalls nach Aufkonzentrierung (Hauptstrom) einer Demineralisierung unterwirft, bei der schwerlösliche Salze gefällt werden,
(c) den demineralisierten Rückstand gegebenenfalls nach weiterer Aufkonzentrierung abkühlt, bis Lactose kristallin ausfällt,
(d) die Lactosekristalle von der Mutterlauge abtrennt und entwässert, um eine erste Menge an Lactose zu erhalten,

Die vorliegende Erfindung ergänzt dieses Standardverfahren um einen diskontinuierlichen Schritt, so dass in Summe ein halb-kontinuierliches Verfahren resultiert.

### Trennung von Proteinen und Lactose

Im ersten Schritt des Verfahrens wird Molke eine proteinreiche und eine lactosereiche Fraktion aufgespalten. Hauptbestandteil der Molke ist unabhängig von ihrer Herkunft Lactose, die mehr als 70 Prozent der Trockenmasse ausmacht. Vom Gewichtsanteil her folgen dann im weiten Abstand Protein und Mineralstoffe. In der Zusammensetzung dieser Stoffklassen liegen neben dem pH-Wert die Unterschiede zwischen den verschiedenen Molkentypen. Typische Zusammensetzungen aus der Literatur sind in Tabelle 1 wiedergegeben:

**Tabelle 1**

| Zusammensetzung verschiedener Molketypen (Angaben in Gew.-%) | | | |
|---|---|---|---|
| **Bestandteil** | **Süssmolke** | **Sauermolke** | **Caseinmolke** |
| Trockenmasse | 6,20 | 5,70 | 6,10 |
| Lactose | 4,80 | 4,60 | 4,70 |
| Proteine | 0,75 | 0,30 | 0,50 |
| Fett | 0,05 | < 0,01 | < 0,01 |
| Asche | 0,60 | 0,80 | 0,90 |
| pH Wert | 6,1 | 4,6 | 4,4 |

Die Süßmolke hat den höchsten Protein- und den geringsten Mineralstoffgehalt. Ihr pH-Wert ist fast neutral. Sie stellt vom gesamten Molkenaufkommen den größten Anteil dar und sie ist der wichtigste Rohstoff für die Herstellung von Molkenpulver und Molkenderivaten. Die Sauermolke und die Caseinmolke haben einen pH-Wert unter 5 und damit einen sauren Geschmack. Ihr Proteingehalt ist im Vergleich zur Süßmolke geringer, dafür enthalten sie aber mehr Mineralstoffe.

Das bevorzugte Trennverfahren stellt die Ultrafiltration (UF) dar, bei der das UF Retentat zur Gewinnung von Proteinen weiterverarbeitet und das UF Permeat für die Gewinnung der Lactose verwendet wird. Dabei unterscheidet man Mikrofiltration, Nanofiltration und Ultrafiltration über den Grad der Abtrennung. Werden Partikel mit der Größe 0,5-0,1 µm abgetrennt, spricht man von Mikrofiltration, sind die Partikel 0,1-0,01 µm groß, dann bezeichnet man es als Ultrafiltration. Typischerweise enthält das UF Retentat etwa 20 Gew.-% Trockenmasse und davon etwa 2 Gew.-% Lactose, der Aschegehalt liegt bei etwa 1 Gew.-%. Das UF Permeat, das zur Herstellung der Lactose weiterverarbeitet wird, weist hingegen eine Trockenmasse von etwa 4,5 bis 5,5 Gew.-% auf, wobei der Gehalt an Lactose bei ca. 4,1 bis 4,6 und der Aschegehalt bei etwa 0,3 bis 0,5 liegt.

### Erste Aufkonzentrierung

Als optionaler, jedoch im Allgemeinen bevorzugter Schritt, wird das UF Permeat aufkonzentriert, wobei man eine Trockenmasse von etwa 10 bis 30 Gew.-% - entsprechend 10 bis 30 °Brix - einstellt. Dies erfolgt vorzugsweise entweder durch Umkehrosmose (RO) oder Nanofiltration (NF).

### Demineralisierung

Das UF Permeat weist - gegebenenfalls nach Aufkonzentrieren - einen Gehalt an Mineralien in der Größenordnung von 1 bis 2 Gew.-% auf. Um die Spezifikation, die bei unter 0,3 Gew.-% liegt, erreichen zu können, werden die Lösungen durch Zugabe von Basen zunächst auf einen annähernd neutralen pH-Wert im Bereich von 6 bis 8 eingestellt und die Mineralien, die im Wesentlichen lösliche Phosphate darstellen mit einer solchen Menge einer Lösung eines wasserlöslichen Calciumsalzes versetzt, dass schwerlösliche Calciumsalze gefällt werden. Zur Einstellung des pH-Wertes und zur Fällung werden NaOH, eine wässrige Zubereitung aus Calciumchlorid und Alkalihydroxid oder Calciumhydroxid einsetzt. Grundsätzlich können zur Einstellung des pH-Wertes auch andere Alkali- oder Erdalkalibasen, wie z.B.-KOH eingesetzt werden. Auch die Natur des Fällungssalzes ist an sich unkritisch, es lassen sich beispielsweise Bariumsalze fällen. Die Verwendung von Calciumsalzen hat indes den Vorteil, dass das Fällungsmittel kostengünstig und die Salze ein sehr niedriges Löslichkeitsprodukt haben, die Fällung also im Wesentlichen vollständig ist. Auch ohne Zugabe von Fällungsmitteln erfolgt die Demineralisierung in Rührkesseln, wobei es sich als vorteilhaft erwiesen hat, eine Temperatur im Bereich von etwa 50 bis 90 und vorzugsweise von etwa 80 °C einzustellen. Die Fällungszeit liegt typischerweise bei etwa 20 bis 120 und vorzugsweise etwa 30 bis 45 min, wobei diese Angaben nur als Anhaltspunkte zu verstehen sind, da niedrigere Temperaturen längere Reaktionszeiten erfordern und umgekehrt. Nach der Fällung werden die Salze abgetrennt, beispielsweise in Separatoren, welche das größere spezifische Gewicht der gefällten Partikel ausnutzen. Es ist aber ebenso möglich, die Abtrennung beispielsweise durch Membranfilter im Rahmen einer weiteren Ultrafiltration im Bereich 5 bis 150 kDa, vorzugsweise 10 bis 50 kDa vorzunehmen.

Der gereinigte Strom enthält jetzt typischerweise etwa 15 bis 20, vorzugsweise etwa 17,5 Gew.-% Lactose, während der Aschegehalt bereits auf etwa 0,8 vermindert worden ist. Falls gewünscht oder erforderlich, kann sich nun eine zweite Demineralisierungsstufe anschließen, bei der man dem vorgereinigten Strom eine Menge an niederen Alkoholen, insbesondere an Ethanol zusetzt, um das Löslichkeitsprodukt der noch enthaltenen Calciumsalze weiter herabzusetzen. Auf diese Weise kann im Bedarfsfall eine weitere Menge an Salzen gefällt und wie oben beschrieben abgetrennt werden.

### Zweite Aufkonzentrierung

Als zweiter optionaler, jedoch im Allgemeinen ebenfalls bevorzugter Schritt, wird der demineralisierte lactosereiche Strom nach Verlassen der Separatoren ein weiteres Mal aufkonzentriert, wobei man einen Feststoffgehalt - im Wesentlichen identisch mit dem Gehalt an Lactose - von etwa 50 bis 70 Gew.-% - entsprechend 40 bis 50 °Brix - einstellt. Dies erfolgt vorzugsweise durch eine Vakuumeindampfung, bei der das Produkt auf vorzugsweise etwa 65 Gew.-% entwässert und gegebenenfalls auch Alkohol aus der Demineralisierungsstufe abgetrennt wird.

### Kristallisation

Anschließend wird der so erhaltene Strom - vorzugsweise nach dem zweiten Aufkonzentrieren - auf eine solche Temperatur abkühlt, dass sich Lactosekristalle aus der Lösung abscheiden. Typische Versuchsbedingungen sind dabei Temperaturen zwischen etwa 5 und 20, vorzugsweise etwa 15 °C und Kristallisationszeiten von etwa 12 bis 48, vorzugsweise etwa 24 h. Es empfiehlt sich, die Lösung mit Lactosekristallen zu impfen, um den Abscheidevorgang zu beschleunigen.

Nachdem sich die Kristalle im Kristallisationsbehälter, beispielsweise einem Rührkessel, abgeschieden haben, werden sie von der Mutterlauge abgetrennt, bei Bedarf mit wässrigem Ethanol oder Wasser gewaschen und erneut entwässert. Anschließend wird mit Hilfe eines Band-, Fließbett oder ähnlichen Trockners bei Temperaturen von etwa 60 °C die Restfeuchte größtenteils entzogen. Die so erhaltene Hauptmenge an Lactose liegt als Monohydrat vor und weist einen Aschegehalt von weniger als 0,3 Gew.-% auf.

### Zusätzliche diskontinuierliche Aufreinigung der Mutterlauge

Vorstehend beschrieben wurde ein an sich bekanntes Verfahren zur Gewinnung von Lactose aus UF Permeat, das kontinuierlich betrieben wird. Das Wesen der Erfindung besteht nun darin, die bei der im letzten Schritt erfolgenden Abtrennung der Lactosekristalle anfallende Mutterlauge, die noch einen Gehalt von etwa 30 Gew.-% Lactose aufweist, separat aufzureinigen und dies in derselben Anlage durchzuführen, in der auch das kontinuierliche Verfahren betrieben wird. Die ökonomischen Vorteile hierfür liegen auf der Hand: Wird die gleiche Anlage verwendet, besteht kein Investitionsbedarf für eine separate zweite Anlage. Dazu ist es lediglich erforderlich, den kontinuierlichen Prozess in Intervallen zu unterbrechen und eine Kampagne, bei der statt des Hauptstroms die Mutterlauge demineralisiert und dann weiterverarbeitet wird, zwischen zu schieben. Die Anlagenkapazität dieses Prozessbereiches sowie die Stapelkapazitäten sind entsprechend zu dimensionieren. Umgekehrt bedeutet dies jedoch nicht, dass die erfinderische Lehre es zwingend erforderlich macht, die gleiche Anlage zu benutzen; wenn es ökonomische Gründe gibt, weswegen die Aufreinigung der Mutterlauge in einer separaten Anlage Sinn macht, so hat dies natürlich auf den Erfolg der Erfindung keinen Einfluss. In beiden Fällen werden natürlich die gleich hohen Lactosequalitäten erhalten, die ein Verschneiden mit dem Hauptprodukt problemlos möglich machen.

Die bevorzugte Ausführungsform der vorliegenden Erfindung sieht indes vor, das kontinuierliche Verfahren um einen diskontinuierlichen Schritt zu ergänzen, so dass in Summe ein halb-kontinuierliches Verfahren resultiert. Dieses zeichnet sich dadurch aus, dass man so viel Mutterlauge sammelt, bis eine Menge erreicht ist, die der Kapazität der Anlagentechnik wie z. B. Kristallisationsbehälter entspricht (z.B. etwa 5 bis 200 Tonnen; das UF Permeat des Hauptstroms, gegebenenfalls nach Konzentrierung, wird zwischenzeitlich in einem Sammeltank gestapelt und währenddessen die Mutterlauge in der gleichen Anlage demineralisiert, aufkonzentriert und den Kristallisationsbehältern zugeführt. Je nach Kapazität der Kristallisationsstufe wird das Konzentrat aus der Mutterlauge separat zu kristalliner Lactose verarbeitet oder mit der Hauptmenge verschnitten. Die hierbei anfallende Mutterlauge 2 sollte verworfen werden um mit dem Konzentrat aus der Demineralisierung ein verkaufsfähiges Produkt zu erhalten. Hier sind aber auch andere Verwertungskanäle realisierbar.

Im Folgenden wird die technische Lehre der Erfindung durch eine Reihe von Ausführungsbeispielen illustriert, ohne dass sie dadurch auf diese Beispiele eingeschränkt wird.

### Beispiele

### Vergleichsbeispiel 1

Ausgangsmaterial für die Gewinnung von Lactose war Molke, die nach Entfettung und Pasteurisierung einer Ultrafiltration (UF) unterworfen wurde. Auf diese Weise wurden zwei Fraktionen erhalten: ein UF Retentat und ein UF Permeat. Das Retentat besaß einen Proteingehalt von 10 bis 20 Gew.-% und enthielt ca. 2 Gew.-% Lactose; der Aschegehalt liegt bei etwa 1 Gew.-%. Das Retentat wurde sprühgetrocknet, um daraus wertvolles Proteinmaterial zu gewinnen.

Die andere Fraktion, das UF Permeat, enthielt etwa 4,7 Gew.-% Lactose und wies einen Aschegehalt von etwa 0,4 Gew.-% auf. Das Permeat wurde einer Umkehrosmose (RO) unterworfen und auf diese Weise auf einen Feststoffgehalt von etwa 20 Gew.-% entsprechend etwa 20 °Brix konzentriert. Anschließend wurde das Konzentrat demineralisiert. Zu diesem Zweck wurde eine solche Menge Natriumhydroxid zugegeben, dass sich ein pH-Wert von 7 einstellte. Anschließend wurde die Lösung für ca. 30 min auf etwa 80 °C erhitzt, wobei schwerlösliches Calciumphosphat ausfiel, das mit Hilfe von Separatoren abgetrennt wurde. Der Rückstand wies einen Lactosegehalt von etwa 5 Gew.-% und einen Aschegehalt von etwa 20 Gew.-% auf.

Die aufgereinigte Lactoselösung wurde als nächstes einer Vakuumeindampfung unterworfen und auf einen Feststoffgehalt von etwa 65 Gew.-% entsprechend etwa 45 °Brix eingestellt. Der Lactosegehalt lag bei etwa 60 bis 62 Gew.-%, der Aschegehalt bei etwa 3 bis 5 Gew.-%. Das Konzentrat verlies den Verdampfer mit etwa 65 °C und wurde in einer Kristallisationsvorrichtung innerhalb von etwa 24 h bis auf 15 °C abgekühlt wird. Die gebildeten Kristalle wurden in eine Siebzentrifuge überführt, in der sie abdekantiert und mit einer Trockenmasse von etwa 90 Gew.-% in einem Trockner entwässert wurden. Die so erhaltene kristalline Lactose wies noch einen Aschegehalt von etwa 0,2 Gew.-% auf. Die bei der Abtrennung der Lactosekristalle zurückbleibende Mutterlauge enthielt noch etwa 20 Gew.-% Lactose und besaß einen Aschegehalt von ca. 15 Gew.-%.

### Vergleichsbeispiel V2

Beispiel V1 wurde wiederholt, die Mutterlauge jedoch im Kreis gefahren und wieder in die Demineralisierungsstufe aufgegeben. Nach Durchlaufen des weiteren Prozesses mit Vaku umeindampfung, Kristallisation und Dekantieren wurde eine zweite Mutterlauge erhalten, die weiterhin ca. 20 Gew.-% Lactose enthielt. Die von Wasser befreiten Lactosekristalle wiesen jedoch einen erhöhten Aschegehalt von 0,25 Gew.-% auf.

### Vergleichsbeispiel V3

Beispiel V2 wurde wiederholt und die Mutterlauge zwei weitere Male im Kreis gefahren. Beim zweiten Durchgang wurde eine Mutterlauge erhalten, die nur noch 18 Gew.-% Lactose enthielt, der Aschegehalt in der kristallisierten Lactose stieg jedoch auf 0,29 Gew.-% an. Beim dritten Durchgang enthielt die Mutterlauge sogar 22 Gew.-% Lactose, der Aschegehalt in der kristallinen Lactose betrug indes 0,32 Gew.-% und war somit nicht mehr spezifikationsgerecht. Die Beispiele V2 und V3 zeigen also, dass eine kontinuierliche Rückführung der Mutterlauge zwar dazu führt, dass die Ausbeute an Lactose deutlich verbessert wird, dies wird jedoch damit erkauft, dass das Produkt nicht mehr die Spezifikation erfüllt.

### Beispiel 1

Beispiel V1 wurde wiederholt. Statt die Mutterlauge in die Demineralisierungsstufe zurückzufahren, wurde sie separat jedoch analog dem Vergleichsbeispiel V1 von Phosphaten befreit, aufkonzentriert, abgekühlt und die abgeschiedenen Kristalle von Wasser befreit. Es wurde eine Mutterlauge erhalten, die nur noch 19 Gew.-% Lactose enthielt, die kristalline Lactose wies jedoch einen Aschegehalt von 0,22 Gew.-% auf und war damit lediglich unwesentlich schlechter als die Lactose, die im Hauptverfahren gewonnen worden war.

### Beispiel 2

Die zweite Mutterlauge aus Beispiel 1 wurde ebenfalls wieder separat aufgearbeitet. Dabei wurde eine dritte Mutterlauge erhalten, die einen Lactosegehalt von 18,5 Gew.-% aufwies. Die resultierenden Lactosekristalle wiesen jedoch ebenfalls wieder einen Aschegehalt von 0,22 Gew.-% auf.

### Beispiel 3

Die dritte Mutterlauge aus Beispiel 2 wurde ebenfalls wieder separat aufgearbeitet. Dabei wurde eine vierte Mutterlauge erhalten, die einen Lactosegehalt von 17 Gew.-% aufwies. Die resultierenden Lactosekristalle wiesen einen Aschegehalt von 0,23 Gew.-% auf. Die Beispiele 1 bis 3 zeigen, dass eine getrennte Aufarbeitung der Mutterlaugen dazu führt, dass die Gesamtausbeute des Verfahrens signifikant verbessert wird, ohne dass der Aschegehalt in der Lactose pro Zyklus ansteigt. In allen Fällen werden Produkte erhalten, die zwar einen marginal höheren Aschegehalt als das Hauptprodukt aufweisen, alle Produkte sind jedoch spezifikationsgerecht und können mit dem Hauptprodukt verschnitten werden.

### Beispiel 4

Beispiel V1 wurde wiederholt. Die erste Mutterlauge wurde gesammelt und sobald ein Volumen von etwa 25 t bevorratet war - entsprechend der Kapazität der Demineralisierungseinheit - wurde der kontinuierliche Aufarbeitungsprozess unterbrochen und das auf etwa 20 °Brix angereicherte UF Permeat in einen Tank gefahren und gesammelt. Stattdessen wurde die Mutterlauge in der Demineralisierungseinrichtung von Phosphat befreit und durchlief die weiteren Schritte der Vakuumeindampfung, Kristallisation, und Trocknung. Es wurde eine zweite Mutterlauge mit einem Lactosegehalt von 25 Gew.-% erhalten, während die so gewonnenen Lactosekristalle einen Aschegehalt von 0,21 Gew.-% *aufwiesen.* Anschließend wurde das im Tank gesammelte aufkonzentrierte UF Permeat wieder in den kontinuierlichen Prozess zurückgeführt. Die dabei anfallende Mutterlauge wurde wieder gesammelt und anschließend wie oben beschrieben weiter verfahren.

## Patentansprüche

1. Verfahren zur Ausbeuteverbesserung bei der Gewinnung von Lactose mit einem Mineralstoffanteil von weniger als 0,3 Gew.-%" bei dem man
(a) Molke einer Ultrafiltration (UF) unterwirft, bei dem ein proteinreiches UF-Retentat und eine lactosereiches UF-Permeat erhalten wird,
(b) das lactosereiche UF-Permeat durch Umkehrosmose (RO) oder Nanofiltration (NF) auf eine Feststoffkonzentration von 10 bis 30 Gew.-% - entsprechend 10 bis 30 °Brix - einstellt,
(c) das so erhaltene Konzentrat einer Demineralisierung unterwirft, bei der schwerlösliche Salze gefällt werden,
(d) den demineralisierten Rückstand nach weiter Aufkonzentrierung abkühlt, bis Lactose kristallin ausfällt,
(e) die Lactosekristalle von der Mutterlauge abtrennt und entwässert, um eine erste Menge an Lactose zu erhalten und schließlich
(f) die Mutterlauge getrennt vom Hauptstrom einer Demineralisierung, Aufkonzentrierung, Abkühlung, Abtrennung der Kristalle und Trocknung unterwirft und die so gewonnene zweite Menge an Lactose mit der ersten Menge vermischt.

2. Verfahren nach Anspruch 1, **dadurch gekennzeichnet, dass** das UF Retentat zur Gewinnung von Proteinen weiterverarbeitet und das UF Permeat für die Gewinnung der Lactose verwendet.

3. Verfahren nach Anspruch 1 und/oder 2, **dadurch gekennzeichnet, dass** man das das UF Permeat durch Zugabe von Basen auf einen pH-Wert im Bereich von 6 bis 8 einstellt und durch Zugabe einer Lösung eines wasserlöslichen Calciumsalzes schwerlösliche Calciumsalze fällt.

4. Verfahren nach Anspruch 3, **dadurch gekennzeichnet, dass** man zur pH-Wert-Einstellung und Fällung entweder eine wässrige Zubereitung aus Calciumchlorid und Alkalihydroxid oder Calciumhydroxid einsetzt.

5. Verfahren nach mindestens einem der Ansprüche 1 bis 4, **dadurch gekennzeichnet, dass** man das UF Permeat über einen Zeitraum von 20 bis 120 Minuten und über einer Temperatur im Bereich von 50 bis 90 °C mit den Calciumverbindungen behandelt.

6. Verfahren nach mindestens einem der Ansprüche 1 bis 5, **dadurch gekennzeichnet, dass** man die schwerlöslichen Salze über Membranen oder Separatoren abtrennt.

7. Verfahren nach mindestens einem der Ansprüche 1 bis 6, **dadurch gekennzeichnet, dass** man die demineralisierte Lactoselösung anschließend von Wasser befreit und auf einen Feststoffgehalt von 50 bis 70 Gew.-% - entsprechend 40 bis 50 °Brix- einstellt.

8. Verfahren nach mindestens einem der Ansprüche 1 bis 7, **dadurch gekennzeichnet, dass** man die aufkonzentrierte Lactoselösung auf eine Temperatur von 5 bis 20 °C abkühlt, so dass sich Lactosekristalle aus der Lösung abscheiden.

9. Verfahren nach mindestens einem der Ansprüche 1 bis 8, **dadurch gekennzeichnet, dass** man die Lactosekristalle von der Mutterlauge abtrennt.

10. Verfahren nach Anspruch 9, dass man die Lactosekristalle entwässert.

11. Verfahren nach mindestens einem der Ansprüche 1 bis 10, **dadurch gekennzeichnet, dass** man das Verfahren halbkontinuierlich durchführt.

12. Verfahren nach Anspruch 11, **dadurch gekennzeichnet, dass** man so viel Mutterlauge sammelt, bis eine Menge erreicht ist, die der Kapazität der Demineralisierungsstufe entspricht, das UF Permeat zwischenzeitlich in einen Sammeltank fährt und gleichzeitig die Mutterlauge in der gleichen Anlage demineralisiert, aufkonzentriert, und abkühlt, die Lactosekristalle entwässert und der Hauptmenge wieder zusetzt und die zweite Mutterlauge wieder solange in einem separaten Tank sammelt, bis eine genügende Menge vorhanden ist, um den Zyklus erneut zu starten, während spätestens nach Abtrennung der Lactosekristalle von der zweiten Mutterlauge die Menge an zwischenzeitlich gelagertem UF Permeat wieder in die Demineralisierungsstufe geführt wird.

## Claims

1. A process for improving the yield in the recovery of lactose with a proportion by weight of less than 0.3% by weight, wherein
(a) whey is subjected to ultrafiltration (UF), wherein a protein-rich UF-retentate and a lactose-rich UF-permeate is obtained,
(b) the lactose-rich UF-permeate is adjusted to a solids concentration of 10 to 30% by weight, corresponding to 10 to 30 °Brix, by reverse osmosis (RO) or nanofiltration (NF),
(c) the thus obtained concentrate is subjected to a demineralization in which poorly soluble salts are precipitated,
(d) the demineralized residue after further concentrating is cooled until lactose precipitates crystalline,
(e) the lactose crystals are separated from the mother liquor and dehydrated to obtain a first quantity of lactose and finally
(f) the mother liquor separated from the main stream is subjected to a demineralization, concentration, cooling, separation of the crystals and drying, and mixing the thus obtained second quantity of lactose is mixed with the first quantity of lactose.

2. A process according to claim 1, **characterized in that** the UF-retentate is further processed for the extraction of proteins and the UF permeate is used for the recovery of the lactose.

3. A process according to claim 1 and/or 2, **characterized in that** the UF-permeate is adjusted to a pH value in the range from 6 to 8 by addition of bases and poorly soluble calcium salts are precipitated by addition of a solution of a water-soluble calcium salt.

4. A process according to claim 3, **characterized in that** an aqueous preparation of calcium chloride and alkali hydroxide or calcium hydroxide is used for the pH adjustment and precipitation.

5. A process according to at least one of Claims 1 to 4, **characterized in that** the UF-permeate is treated with the calcium compounds over a period of 20 to 120 minutes and over a temperature in the range of 50 to 90 °C.

6. A process according to at least one of Claims 1 to 5, **characterized in that** the poorly soluble salts are separated off via membranes or separators.

7. A process according to at least one of Claims 1 to 6, **characterized in that** the demineralized lactose solution is subsequently freed from water and adjusted to a solids content of 50 to 70% by weight, corresponding to 40 to 50 ° Brix.

8. A process according to at least one of Claims 1 to 7, **characterized in that** the concentrated lactose solution is cooled to a temperature of 5 to 20 °C so that lactose crystals are separated from the solution.

9. A process according to at least one of Claims 1 to 8, **characterized in that** the lactose crystals are separated from the mother liquor.

10. A process according to claim 9, wherein the lactose crystals are dehydrated.

11. A process according to at least one of Claims 1 to 10, **characterized in that** the process is carried out in a semi-continuous manner.

12. A process according to claim 11, **characterized in that** so much mother liquor is collected until an amount is reached which corresponds to the capacity of the demineralization stage, the UF-permeate meanwhile runs into a collection tank and at the same time, the mother liquor in the same plant is demineralized, concentrated, and cooled, the lactose crystals are dehydrated and the main quantity is re-added and the second mother liquor is recollected in a separate tank until a sufficient quantity is obtained to restart the cycle, while at the latest after separation of the lactose crystals from the second mother liquor the amount of UF permeate, which has been stored in the meantime, is fed back into the demineralization stage.

## Revendications

1. Procédé d'amélioration du rendement lors de la production de lactose ayant une proportion de matières minérales de moins de 0,3 % en poids, selon lequel
(a) le petit lait est soumis à une ultrafiltration (UF), lors de laquelle un rétentat d'UF riche en protéines et un perméat d'UF riche en lactose sont obtenus,
(b) le perméat d'UF riche en lactose est ajusté par osmose inverse (RO) ou nanofiltration (NF) à une concentration en solides de 10 à 30 % en poids, correspondant à 10 à 30 °Brix,
(c) le concentré ainsi obtenu est soumis à une déminéralisation, lors de laquelle des sels difficilement solubles sont précipités,
(d) le résidu déminéralisé est refroidi après une concentration supplémentaire, jusqu'à la précipitation de lactose sous forme cristalline,
(e) les cristaux de lactose sont séparés de la liqueur mère et déshydratés pour obtenir une première quantité de lactose, et enfin
(f) la liqueur mère est soumise séparément du courant principal à une déminéralisation, une concentration, un refroidissement, une séparation des cristaux et un séchage, et la deuxième quantité de lactose ainsi obtenue est mélangée avec la première quantité.

2. Procédé selon la revendication 1, **caractérisé en ce que** le rétentat d'UF est transformé pour l'obtention de protéines et le perméat d'UF est utilisé pour l'obtention du lactose.

3. Procédé selon la revendication 1 et/ou 2, **caractérisé en ce que** le perméat d'UF est ajusté à un pH dans la plage allant de 6 à 8 par ajout de bases et des sels de calcium difficilement solubles sont précipités par ajout d'une solution d'un sel de calcium soluble dans l'eau.

4. Procédé selon la revendication 3, **caractérisé en ce qu'**une préparation aqueuse de chlorure de calcium et d'un hydroxyde alcalin ou de l'hydroxyde de calcium est utilisé pour l'ajustement du pH et la précipitation.

5. Procédé selon au moins l'une quelconque des revendications 1 à 4, **caractérisé en ce que** le perméat d'UF est traité pendant une durée de 20 à 120 minutes et à une température dans la plage allant de 50 à 90 °C avec les composés de calcium.

6. Procédé selon au moins l'une quelconque des revendications 1 à 5, **caractérisé en ce que** les sels difficilement solubles sont séparés par des membranes ou des séparateurs.

7. Procédé selon au moins l'une quelconque des revendications 1 à 6, **caractérisé en ce que** la solution de lactose déminéralisée est ensuite débarrassée de l'eau et ajustée à une teneur en solides de 50 à 70 % en poids, correspondant à 40 à 50 °Brix.

8. Procédé selon au moins l'une quelconque des revendications 1 à 7, **caractérisé en ce que** la solution de lactose concentrée est refroidie à une température de 5 à 20 °C, de sorte que des cristaux de lactose se séparent de la solution.

9. Procédé selon au moins l'une quelconque des revendications 1 à 8, **caractérisé en ce que** les cristaux de lactose sont séparés de la liqueur mère.

10. Procédé selon la revendication 9, **caractérisé en ce que** les cristaux de lactose sont déshydratés.

11. Procédé selon au moins l'une quelconque des revendications 1 à 10, **caractérisé en ce que** le procédé est réalisé de manière semi-continue.

12. Procédé selon la revendication 11, **caractérisé en ce que** de la liqueur mère est collectée jusqu'à atteindre une quantité qui correspond à la capacité de l'étape de déminéralisation, le perméat d'UF est entre temps acheminé dans une cuve de collecte et, simultanément, la liqueur mère est déminéralisée, concentrée et refroidie dans la même unité, les cristaux de lactose sont déshydratés et rajoutés à la quantité principale, et la deuxième liqueur mère est de nouveau collectée dans une cuve séparée jusqu'à ce qu'une quantité suffisante soit présente pour démarrer de nouveau le cycle, tandis qu'au plus tard après la séparation des cristaux de lactose de la deuxième liqueur mère, la quantité de perméat d'UF entreposée entre temps est réintroduite dans l'étape de déminéralisation.
